# EUROPEAN PATENT APPLICATION

(11) **EP 2 589 396 A1**
(43) Date of publication of application: **08.05.2013**
(21) Application number: 11800777.2
(22) Date of filing: 27.06.2011
(51) Int. Cl.: A61M 5/14, A61M 39/02

(54) **TRANSFUSION TUBE AND TRANSFUSION TUBE SET**

(30) Priority: 29.06.2010 JP 2010148281
(71) Applicant: Terumo Kabushiki Kaisha, Tokyo 151-0072 (JP)
(72) Inventor: IMAI, Masaomi, Ashigarakami-gun Kanagawa 259-0151 (JP)
(74) Representative: TBK
(86) International application number: PCT/JP2011/064656
(87) International publication number: WO 2012/002313

(57) **Abstract**

A transfusion tube set (100) includes: a transfusion tube (1); and a transfusion tube (1a) which does not have a filter (26) and a transfusion tube (1b) which has the filter (26), wherein the transfusion tubes (1a) and (1b) are detachable from the transfusion tube (1). The transfusion tube (1) includes a tube (21) and a reservoir needle (22) provided at a upstream end of the tube (21), and a drip tube (23), a roller clamp (24), an upstream connector (3a) and a downstream connector (3b) are provided downstream of the reservoir needle (22) of the tube (21). A filter mesh (12) is disposed at the upstream end of a main tube (31) of the downstream connector (3b).

## Description

### Technical Field

The present invention relates to a transfusion tube and a transfusion tube set.

### Background Art

There have been known transfusion tubes and transfusion tube sets which are used in performing transfusion into a patient.
The transfusion tubes include in-line filtered transfusion tubes equipped with a filter which permits liquid to pass therethrough but prevents bacteria from passing therethrough and in-line filterless transfusion tubes not equipped with the filter. At the time of administering a medicinal liquid into a patient, the in-line filtered transfusion tube and the in-line filterless transfusion tube are used through preliminary selection according to a kind and characteristic properties of the medicinal liquid to be administered (refer to, for example, Patent Document 1). Besides, at the time of using the in-line filterless transfusion tube, the line is provided therein with a filter mesh for removing a foreign matter, such as glass fragments arising from an ampoule or rubber fragments arising from a vial, present in the medicinal liquid flowing through the line.

Here, in the case of using the in-line filtered transfusion tube, the line is not equipped with the filter mesh. This is because the filter having a filter mesh function unnecessitates the filter mesh, and because arrangement of both the filter and the filter mesh in the line leads to an extremely high flow resistance during the flow of the medicinal liquid through the line.

In addition, in the case where a medicine contained in the medicinal liquid to be administered will be adsorbed on the filter, the in-line filtered transfusion tube cannot be used.

Meanwhile, in the case of a conventional transfusion tube set, an in-line filtered transfusion tube and an in-line filterless transfusion tube are put to preliminary selection before use. For example, in a combination chemotherapy of cancer wherein a plurality of carcinostatic agents are administered sequentially, the in-line filtered transfusion tube and the in-line filterless transfusion tube may have to be exchanged during the treatment depending on a carcinostatic agent to be administered. This exchange of transfusion tube is carried out, for example, by a process in which a puncture needle provided at an end of the in-line filtered transfusion tube is withdrawn from the patient and thereafter a puncture needle provided at an end of the in-line filterless transfusion tube is made to puncture the patient, or by a reverse process thereof. Therefore, at the time of the exchange of transfusion tube, the carcinostatic agent remaining in the line may leak out of the puncture needle and a health care staff may be exposed to the carcinostatic agent.

Patent Document 1: Japanese Patent Laid-Open No. S64-16843

### Disclosure of Invention

It is an object of the present invention to provide a transfusion tube and a transfusion tube set which ensure that a filtered transfusion tube and a filterless transfusion tube can be attached and detached safely, without touching a liquid to be administered, and which make possible a configuration such that a filter member is not intermediately disposed in a flow path when the filtered transfusion tube is used, whereas the filter member is intermediately disposed in the flow path when the filterless transfusion tube is used.
In order to attain the above object, according to the present invention, there is provided a transfusion tube including:
a tube constituting a flow path for liquid containing a foreign matter;
a filter member which is provided at an intermediate portion of the flow path and which removes the foreign matter present in the liquid flowing through the flow path;
an upstream connector provided in the flow path upstream of the filter member; and
a downstream connector provided in the flow path downstream of the filter member,
wherein the transfusion tube is configured so that a filtered transfusion tube, which is used during transfusion and which has a filter permitting the liquid to pass therethrough but preventing bacteria from passing therethrough, is connected to the downstream connector, and whereas a filterless transfusion tube which is used during the transfusion and which has no the filter, is connected to the upstream connector.

In the transfusion tube according to the present invention, preferably,
the upstream connector has a branch tube projected to a lateral side of the tube, and the branch tube being provided at an end portion thereof with a connection port to which the filterless transfusion tube is to be connected, and
wherein the downstream connector has a branch tube projected to a lateral side of the tube, and the branch tube being provided at an end portion thereof with a connection port to which the filtered transfusion tube is to be connected.

In the transfusion tube according to the present invention, preferably, the filter mesh is provided downstream of the branch tube of the upstream connector, or upstream of the branch tube of the downstream connector.

In the transfusion tube according to the present invention, preferably, the filterless transfusion tube and the filtered transfusion tube are each for use in administering a carcinostatic agent.

In addition, in order to attain the above object, according to the present invention, there is provided a transfusion tube set including:
the transfusion tube according to the present invention;
the filtered transfusion tube which is used during the transfusion, and which has a tube constituting a flow path for the liquid and the filter provided at an intermediate portion of the flow path and permitting the liquid to pass therethrough but preventing the bacteria from passing therethrough; and
the filterless transfusion tube which is used during the transfusion, and which has a tube constituting a flow path for the liquid and no the filter,
wherein the filtered transfusion tube is connected to the downstream connector, and the filterless transfusion tube is connected to the upstream connector.

In the transfusion tube set according to the present invention, preferably,
the filterless transfusion tube has a filterless transfusion tube side connector which is connected to the upstream connector, and
the filtered transfusion tube has a filtered transfusion tube side connector which is connected to the downstream connector,
wherein one connector of the upstream connector and the filterless transfusion tube side connector includes a tubular first connector main body having an opening to insert to the other connector at a distal end of the first connector main body, a hollow needle provided in a lumen part of the first connector main body so as to communicate with the flow path and having an opening at a distal portion of the hollow needle, a first sealing member having a first pierceable part pierceable by the hollow needle and formed from an elastic material to seal off the lumen part of the first connector main body, and a biasing member for biasing the first pierceable part in a distal direction,
wherein the other connector includes a tubular second connector main body of which distal end communicates with the flow path, and a second sealing member provided at a proximal end of the second connector main body, having a second pierceable part making close contact with the first pierceable part when inserted into the first connector main body, and formed from an elastic material to seal off a lumen part of the second connector main body,
wherein the one connector is provided with operation restraining means by which at the time of inserting the other connector into the one connector, an inserting operation is once restrained in its course and restarting of the inserting operation is enabled when the restraint is removed, and, at the time of withdrawing the other connector from the one connector, a withdrawing operation is once restrained in its course and restarting of the withdrawing operation is enabled when the restraint is removed,
in a process of inserting the other connector into the one connector, the inserting operation is once restrained by the operation restraining means when the first pierceable part and the second pierceable part are brought into a close contact state, and, when the inserting operation is restarted by removing the restraint, the first pierceable part and the second pierceable part in the close contact state are pierced through by the hollow needle, and the opening of the hollow needle is located on the distal side relative to the second pierceable part, to be exposed to the inside of the lumen part of the second connector main body, and
wherein in a process of withdrawing the other connector from the one connector, the withdrawing operation is once restrained by the operation restraining means when the opening of the hollow needle is located on the proximal side relative to the first pierceable part in the close contact state, and, when the withdrawing operation is restarted by removing the restraint, the first pierceable part and the second pierceable part are spaced away from each other.

In the transfusion tube set according to the present invention, preferably,
the filterless transfusion tube has a filterless transfusion tube side connector which is connected to the upstream connector, and
the filtered transfusion tube has a filtered transfusion tube side connector which is connected to the downstream connector,
wherein one connector of the downstream connector and the filtered transfusion tube side connector includes a tubular third connector main body having an opening to insert the other connector at a distal end of the third connector main body, a hollow needle provided in a lumen part of the third connector main body so as to communicate with the flow path and having an opening at a distal portion of the hollow needle, a third sealing member having a third pierceable part pierceable by the hollow needle and formed from an elastic material to seal off the lumen part of the third connector main body, and a biasing member for biasing the third pierceable part in a distal direction,
wherein the other connector includes a tubular fourth connector main body of which distal end communicates with the flow path, and a fourth sealing member provided at a proximal end of the fourth connector main body, having a fourth pierceable part making close contact with the third pierceable part when inserted into the third connector main body, and formed from an elastic material to seal off a lumen part of the fourth connector main body,
wherein the one connector is provided with operation restraining means by which at the time of inserting the other connector into the one connector, an inserting operation is once restrained in its course and restarting of the inserting operation is enabled when the restraint is removed, and, at the time of withdrawing the other connector from the one connector, a withdrawing operation is once restrained in its course and restarting of the withdrawing operation is enabled when the restraint is removed,
wherein in a process of inserting the other connector into the one connector, the inserting operation is once restrained by the operation restraining means when the third pierceable part and the fourth pierceable part are brought into a close contact state, and, when the inserting operation is restarted by removing the restraint, the third pierceable part and the fourth pierceable part in the close contact state are pierced through by the hollow needle, and the opening of the hollow needle is located on the distal side relative to the fourth pierceable part, to be exposed to the inside of the lumen part of the fourth connector main body, and
wherein in a process of withdrawing the other connector from the one connector, the withdrawing operation is once restrained by the operation restraining means when the opening of the hollow needle is located on the proximal side relative to the third pierceable part in the close contact state, and, when the withdrawing operation is restarted by removing the restraint, the third pierceable part and the fourth pierceable part are spaced away from each other.

In the transfusion tube according to the present invention, preferably, the filter member is a filter mesh which is mesh-like in form.

In the transfusion tube according to the present invention, preferably, a mesh size of the filter mesh is 50 to 800 µm.

In the transfusion tube according to the present invention, preferably, the upstream connector and the downstream connector are so configured that they can be distinguished from each other on a visual basis.

The transfusion tube according to the present invention, preferably, has a connection section which is provided on the upstream side of the upstream connector and to which a liquid storage container capable of storing a liquid is connected.

### Brief Description of Drawings

[FIG. 1]

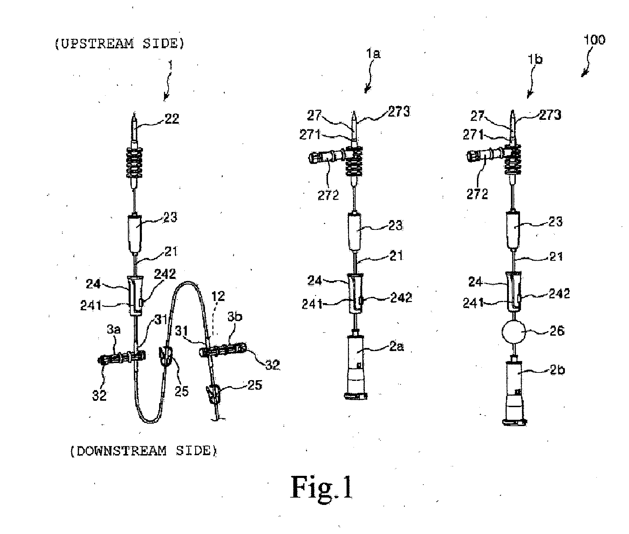

   FIG. 1 shows a plan view showing a first embodiment of a transfusion tube set according to the present invention.
[FIG. 2]

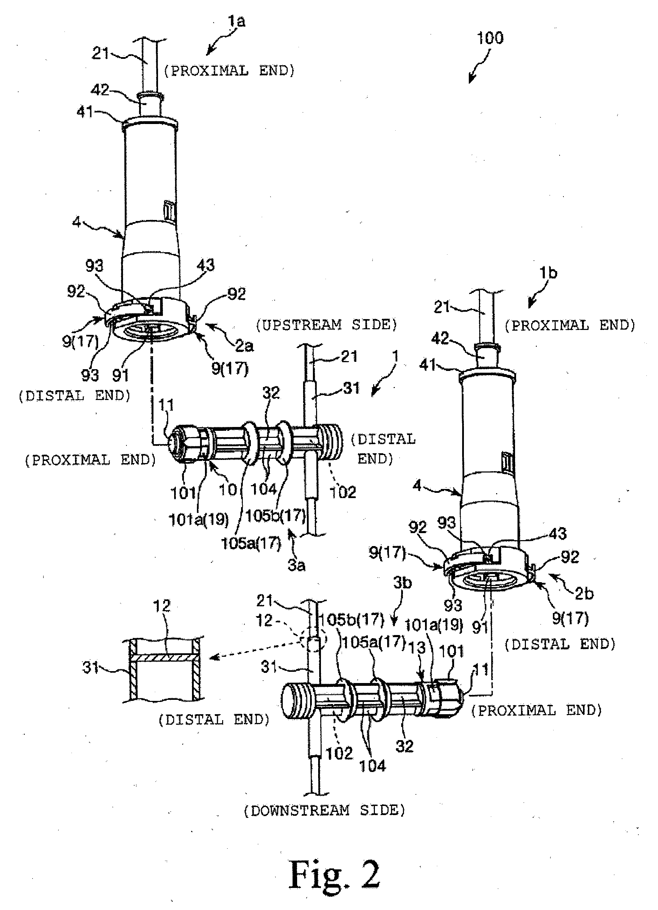

   FIG. 2 shows perspective views showing an upstream connector, a downstream connector, a filterless transfusion tube side connector and a filtered transfusion tube side connector of the transfusion tube set shown in FIG. 1.
[FIG. 3]

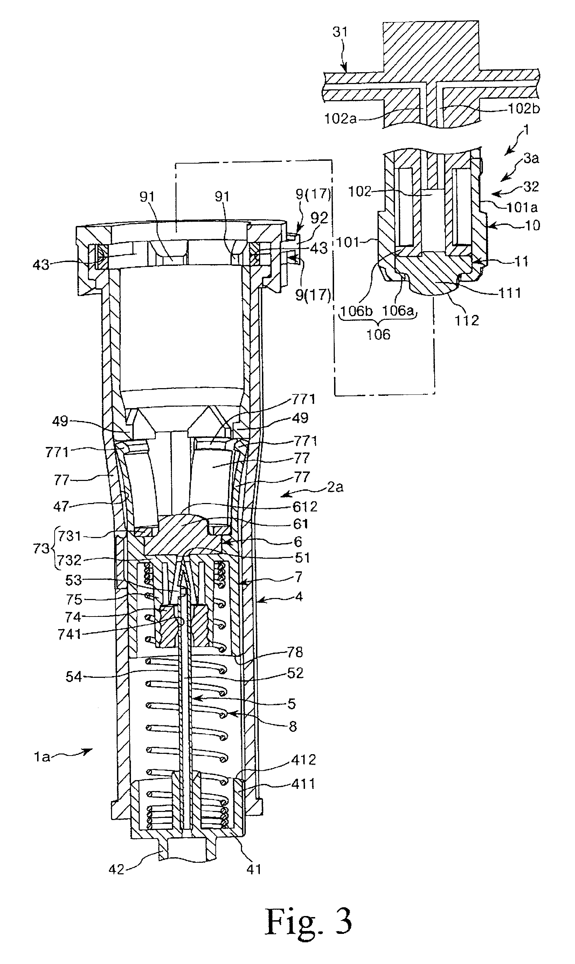

   FIG. 3 is a longitudinal sectional view illustrating a process until the filterless transfusion side connector and the upstream connector in the transfusion tube set shown in FIG. 1 are put into a connected state.
[FIG. 4]

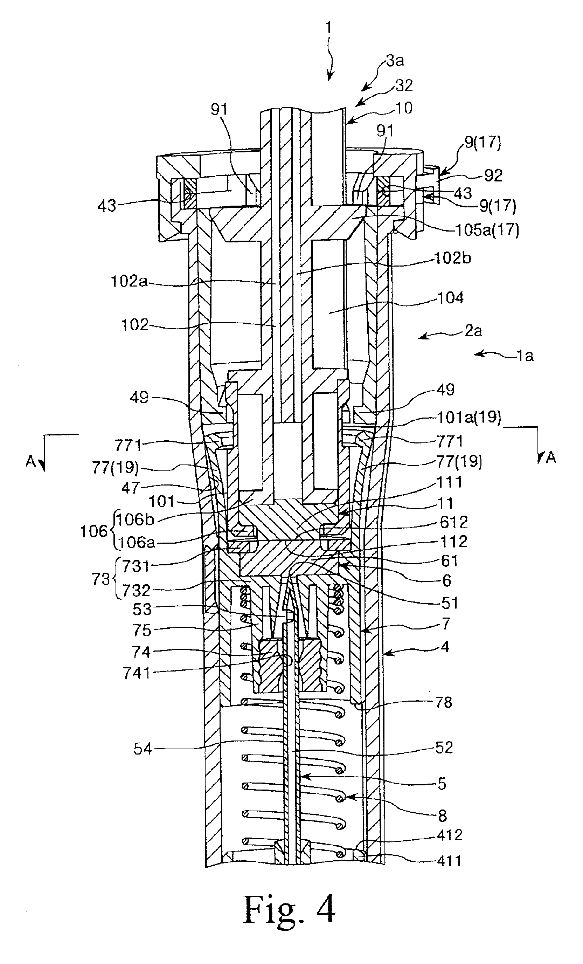

   FIG. 4 is a longitudinal sectional view illustrating the process until the filterless transfusion tube side connector and the upstream connector in the transfusion tube set shown in FIG. 1 are put into the connected state.
[FIG. 5]

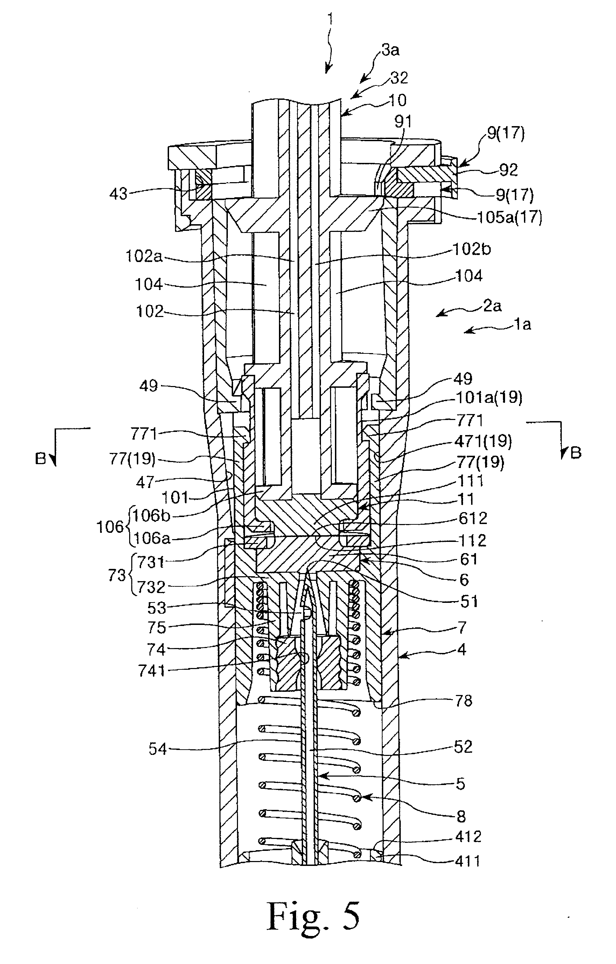

   FIG. 5 is a longitudinal sectional view illustrating the process until the filterless transfusion tube side connector and the upstream connector in the transfusion tube set shown in FIG. 1 are put into the connected state.
[FIG. 6]

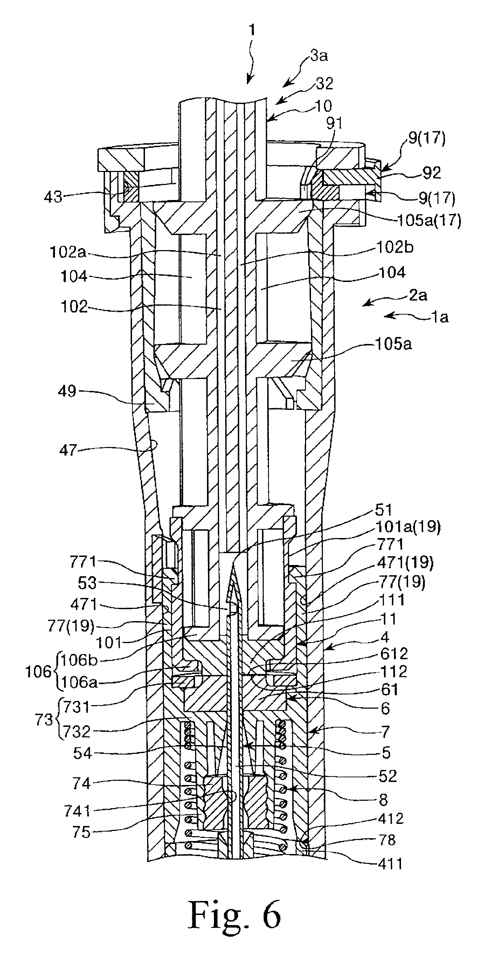

   FIG. 6 is a longitudinal sectional view illustrating the process until the filterless transfusion tube side connector and the upstream connector in the transfusion tube set shown in FIG. 1 are put into the connected state.
[FIG. 7]

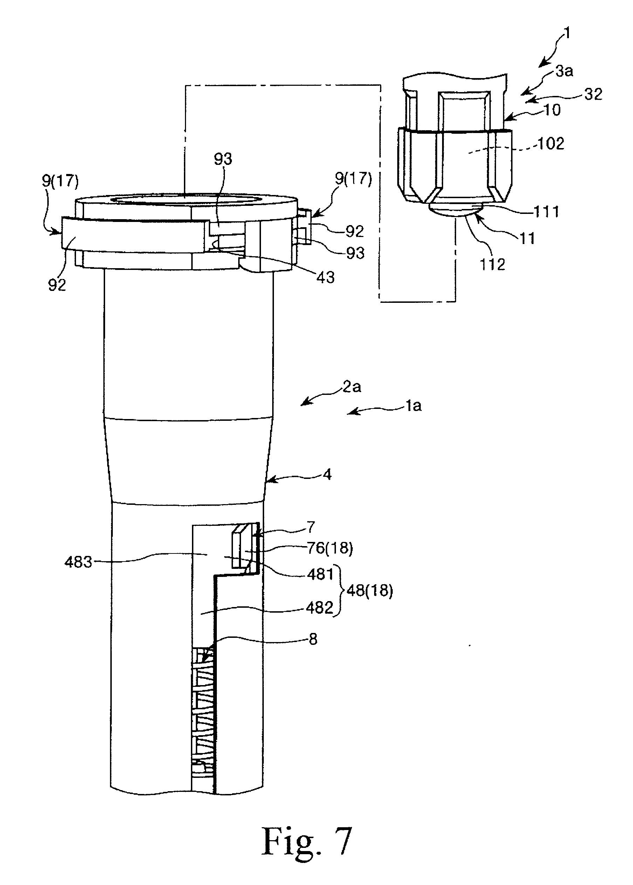

   FIG. 7 is a perspective view (corresponding to FIG. 3) illustrating the process until the filterless transfusion tube side connector and the upstream connector in the transfusion tube set shown in FIG. 1 are put into the connected state.
[FIG. 8]

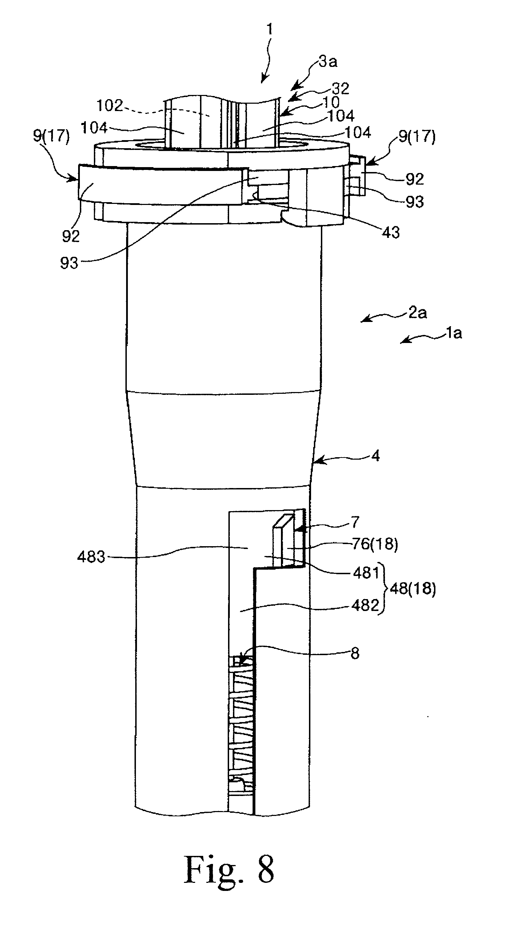

   FIG. 8 is a perspective view (corresponding to FIG. 4) illustrating the process until the filterless transfusion tube side connector and the upstream connector in the transfusion tube set shown in FIG. 1 are put into the connected state.
[FIG. 9]

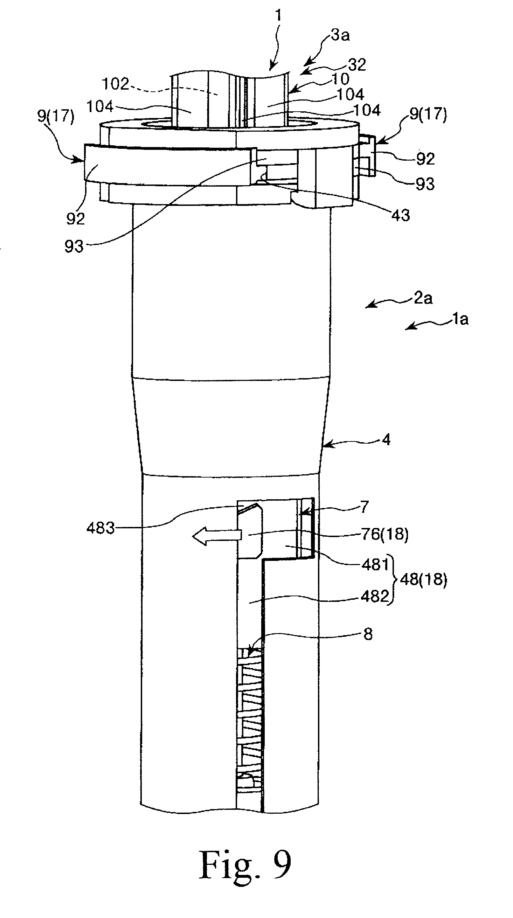

   FIG. 9 is a perspective view (corresponding to FIG. 5) illustrating the process until the filterless transfusion tube side connector and the upstream connector in the transfusion tube set shown in FIG. 1 are put into the connected state.
[FIG. 10]

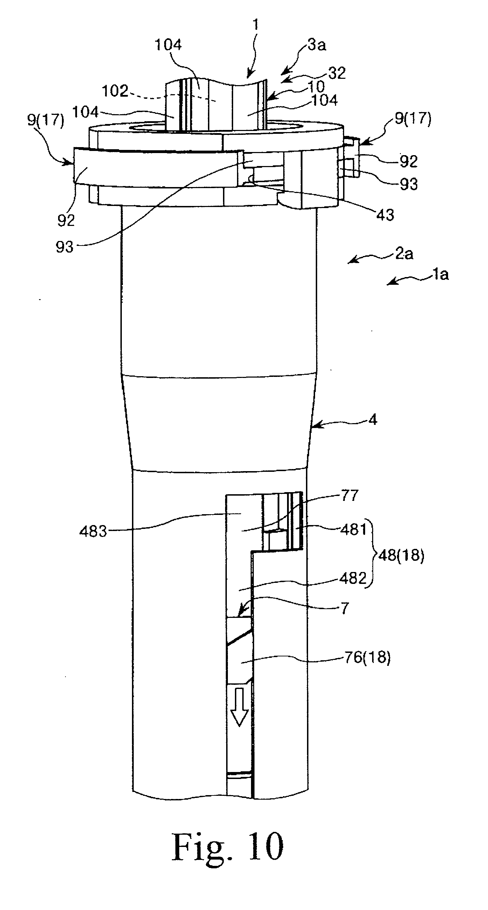

   FIG. 10 is a perspective view (corresponding to FIG. 6) illustrating the process until the filterless transfusion tube side connector and the upstream connector in the transfusion tube set shown in FIG. 1 are put into the connected state.
[FIG. 11]

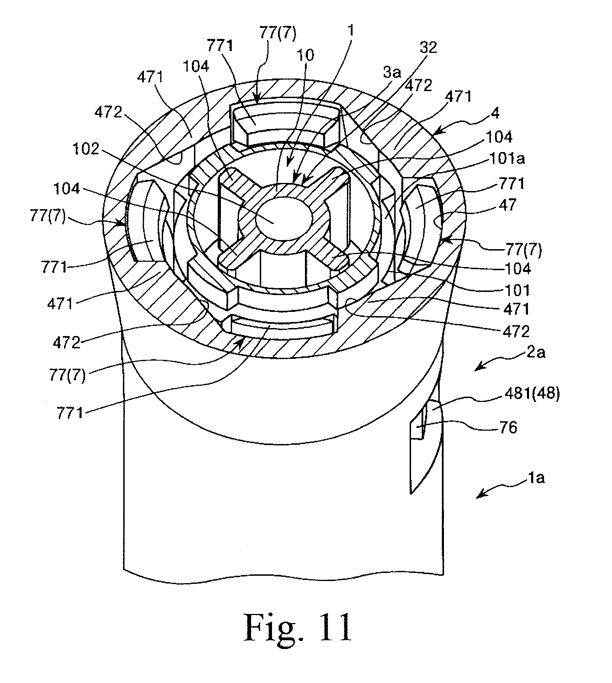

   FIG. 11 is a sectional view taken along line A-A of FIG. 4.
[FIG. 12]

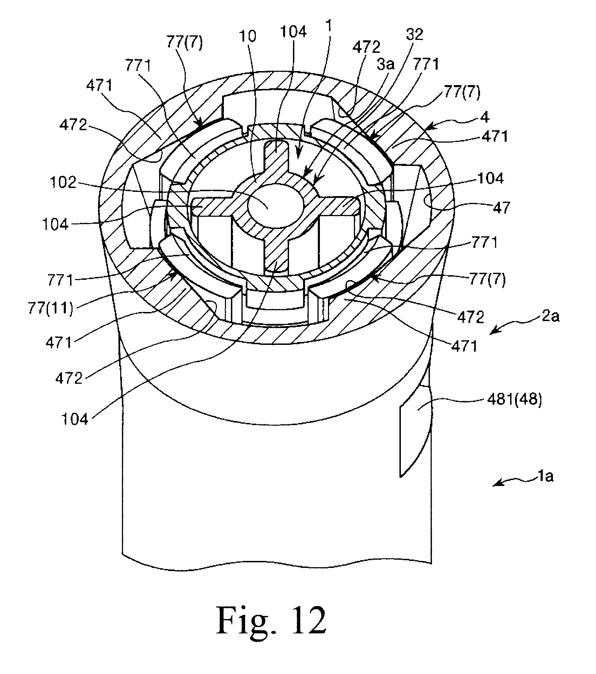

   FIG. 12 is a sectional view taken along line B-B of FIG. 5.
[FIG. 13]

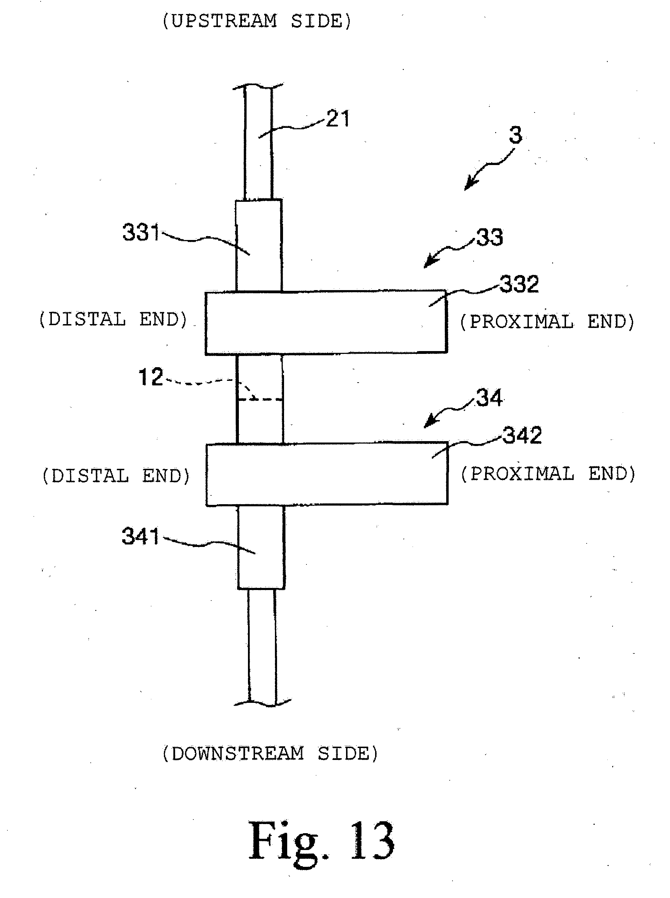

   FIG. 13 is a plan view schematically showing a connector for a transfusion tube in a second embodiment of a transfusion tube set according to the present invention.

### Modes for Carrying Out the Invention

Now, a transfusion tube and a transfusion tube set according to the present invention will be described in detail below, based on preferred embodiments shown in the attached drawings.

### <First Embodiment>

FIG. 1 shows a plan view showing a first embodiment of a transfusion tube set according to the present invention; FIG. 2 shows perspective views showing an upstream connector, a downstream connector, a filterless transfusion tube side connector and a filtered transfusion tube side connector of the transfusion tube set shown in FIG. 1; FIGS. 3 to 6 are longitudinal sectional views illustrating a process until the filterless transfusion tube side connector and the upstream connector in the transfusion tube set shown in FIG. 1 are put into a connected state; FIGS. 7 to 10 are perspective views (corresponding respectively to FIGS. 3 to 6) illustrating the process until the filterless transfusion tube side connector and the upstream connector in the transfusion tube set shown in FIG. 1 are put into the connected state; FIG. 11 is a sectional view taken along line A-A of FIG 4; and FIG. 12 is a sectional view taken along line B-B of FIG. 5.

Incidentally, in the following description, the upper side in each of FIGS. 1 and 2 will be referred to as "upstream (side)," and the lower side as "downstream (side)."

In addition, in regard of each of connectors in FIG. 2, as indicated by parenthesized reference signs in the figure, a filterless transfusion tube side connector 2a and a filtered transfusion tube side connector 2b will be described by referring to the upper side as "proximal (end)" and the lower side as "distal (end)"; a branch tube 32 of an upstream connector 3a will be described by referring to the left side as "proximal (end)" and the right side as "distal (end)"; and a branch tube 32 of a downstream connector 3b will be described by referring to the right side as "proximal (end)" and the left side as "distal (end)." Besides, in FIG. 2, a section of a part surrounded by a broken line is shown.

In addition, the upper side in each of FIGS. 3 to 12 will be referred to as "distal" or "upper" or "upward," and the lower side as "proximal" or "lower" or "downward," in the following description.

A transfusion tube set 100 shown in these figures is a device by which various liquids can be administered into a patient. The transfusion tube set 100 includes a transfusion tube 1 constituting a main line, and transfusion tubes 1a and 1b which can be attached to and detached from the transfusion tube 1 and constitute sublines branched from the main line. The transfusion tube 1b is a filtered transfusion tube equipped with a filter 26 which permits liquid to pass therethrough but prevents bacteria from passing therethrough, whereas the transfusion tube 1a is a filterless transfusion tube which is not equipped with the filter.

The liquid to be administered from the transfusion tube set 100 includes all liquids which can be administered into a living body, such as various medicinal liquids and transfusion preparations such as electrolytes for correction, physiological saline, dextrose in water (DW), Ringer solution, and total parenteral nutrition (TPN).

In addition, a kind of the medicine in the medicinal liquid is not specifically restricted, but may be any of such medicines as a sedative, an intravenous anesthetic, an anesthetic analgesic, a local anesthetic, a nondepolarizing muscle relaxant, a vasopressor, a depressor, a coronary vasodilator, a diuretic, an antiarrhythmic, a bronchodilating agent, a hemostatic agent, vitamin preparations, an antibiotic, a carcinostatic agent, and an antiemetic.

Here, in this embodiment, description will be made of a case wherein one or both of the transfusion tubes 1a and 1b are connected to the transfusion tube 1, and a medicinal liquid containing a medicine which is dangerous if erroneously contacted by a health care staff, specifically, the medicinal liquid containing the carcinostatic agent is administered via the transfusion tube 1a or the transfusion tube 1b. In addition, description will be made of a case wherein a transfusion preparation or the like is administered via the transfusion tube 1, in the condition where none of the transfusion tubes 1a and 1b is connected to the transfusion tube 1 or in the condition where either one or both of the transfusion tubes 1a and 1b are connected to the transfusion tube 1. Incidentally, in administering the carcinostatic agent, the antiemetic or the like can be administered by the transfusion tube 1 prior to or simultaneously with the administration of the carcinostatic agent, for preventing or restraining side effects of the carcinostatic agent.

As shown in FIG. 1, the transfusion tube 1 includes a tube 21 which is flexible and constitutes a flow path for liquid during transfusion, a reservoir needle 22 having a sharp needlepoint and provided at the upstream end of the tube 21, as a connection section to which a liquid storage container capable of storing a liquid is connected, and a connector (not shown) provided at the downstream end of the tube 21. The connector is connected to a connector connected to a catheter or an indwelling needle set indwelling in a patient's blood vessel. Incidentally, a member provided at the downstream end of the tube 21 is not restricted to the connector but may be, for example, a puncture needle by which the patient is to be punctured.

In performing the transfusion, the reservoir needle 22 of the transfusion tube 1 pierces a rubber stopper (not shown) of a bag in which a medicinal liquid containing a predetermined medicine or a transfusion preparation or the like is stored. As a result, the bag and the transfusion tube 1 are connected to each other through the reservoir needle 22.

In addition, at intermediate portions of the tube 21, specifically, at positions downstream of the reservoir needle 22 of the tube 21, there are provided a drip chamber 23, a roller clamp 24 as flow rate control means for controlling a flow rate of the liquid during the transfusion, the upstream connector 3a and the downstream connector 3b, in this order from the upstream side toward the downstream side. Besides, if required, a clamp or clamps 25 for opening and closing the flow path in the tube 21 are disposed at a predetermined part or parts of the tube 21. Incidentally, the upstream connector 3a and the downstream connector 3b will be described in detail later.

The drip chamber 23 is disposed in the vicinity of the reservoir needle 22. The drip chamber 23 makes it possible to visually check the flow rate of the liquid during the transfusion.

The roller clamp 24 is composed of a clamp main body 241 and a roller 242 so disposed as to be movable relative to the clamp main body 241. The tube 21 is clamped between an outer circumferential surface of the roller 242 and a bottom surface, inclined at a predetermined angle, of the clamp main body 241. With the roller 242 moved relative to the clamp main body 241, a degree of clamping (nipping) the tube 21 is varied, whereby the flow rate of the liquid is controlled.

Specifically, when the roller 242 is moved in a predetermined direction, the degree of clamping (nipping) the tube 21 is increased, whereby the flow rate of the liquid is lowered. When the roller 242 is moved to a final position, a lumen of the tube 21 is closed, resulting in that the liquid does not flow.

On the other hand, when the roller 242 is moved in a reverse direction to the above, the degree of clamping (nipping) the tube 21 is decreased, whereby the flow rate of the liquid is enhanced. When the roller 242 is moved to the final position, the lumen of the tube 21 is fully opened, resulting in that the flow rate of the liquid is maximized.

The transfusion tube 1a includes a tube 21 which is flexible and constitutes a flow path for liquid during the transfusion, a connector 27 provided at the upstream end of the tube 21, and the filterless transfusion tube side connector 2a provided at the downstream end of the tube 21. Incidentally, the filterless transfusion tube side connector 2a will be described in detail later.

The connector 27 has a main tube 271 and a branch tube 272 branched from the main tube 271. The main tube 271 is disposed coaxially with the tube 21, and is connected to the upstream end of the tube 21. In addition, a reservoir needle section 273 having a sharp needlepoint is formed an upstream side of the main tube 271, as a connection section to which a liquid storage container capable of storing a liquid is connected. Besides, the branch tube 272 is projecting to a lateral side of the main tube 271, in other words, to a lateral side of the tube 21.

In addition, the main tube 271 is provided therein with two independent lumens each of which constitutes a flow path for liquid. One of the lumens is formed to extend from a distal portion to a proximal portion of the main tube 271, is opening at its one end into a distal portion of the reservoir needle section 273, and communicates at its other end with the flow path in the tube 21. Besides, the other of the lumens is formed to extend from the distal portion of the main tube 271 to a part of the branch tube 272, is opening at its one end into the distal portion of the reservoir needle section 273, and communicates at its other end with the lumen of the branch tube 272.

In addition, at intermediate portions of the tube 21, specifically, at those portions of the tube 21 which are between the connector 27 and the filterless transfusion tube side connector 2a, a drip chamber 23 and a roller clamp 24 as flow rate control means for controlling the flow rate of liquid during the transfusion are provided in this order from the upstream side toward the downstream side. Besides, if required, a clamp or clamps (not shown) for opening and closing the flow path in the tube 21 are disposed at a predetermined part or parts of the tube 21. Incidentally, the transfusion tube 1a is not provided with any filter that permits liquid to pass therethrough but prevents bacteria from passing therethrough.

At the time of the transfusion, the reservoir needle section 273 of the connector 27 of the transfusion tube 1a is made to pierce a rubber stopper of a bag (not shown) in which a medicinal liquid containing a carcinostatic agent is reserved. As a result, the bag and the transfusion tube 1a are connected to each other through the reservoir needle section 273. Incidentally, the medicinal liquid may be preliminarily reserved in the bag, or the medicinal liquid may be stored into the bag afterwards.

The transfusion tube 1b includes a tube 21 which is flexible and constitutes a flow path for liquid during the transfusion, a connector 27 provided at the upstream end of the tube 21, and a filtered transfusion tube side connector 2b provided at the downstream end of the tube 21. Incidentally, the filtered transfusion tube side connector 2b will be described in detail later. In addition, the configuration of the connector 27 is the same as that of the connector 27 of the above-described transfusion tube 1a, and, therefore, the description of the configuration is omitted here.

Besides, at intermediate portions of the tube 21, specifically, at those portions of the tube 21 which are between the connector 27 and the filtered transfusion tube side connector 2b, there are provided a drip chamber 23, a roller clamp 24 as flow rate control means for controlling the flow rate of liquid during the transfusion, and the filter 26 which permits liquid to pass therethrough but prevents bacteria from passing therethrough, in this order from the upstream side toward the downstream side. In addition, if required, a clamp or clamps (not shown) for opening and closing the flow path in the tube 21 are disposed at a predetermined part or parts of the tube 21.

Besides, a mesh size of the filter 26 is not particularly limited, insofar as it is at such a level as to permit the liquid to pass through the filter 26 but to prevent the bacteria from passing through the filter 26. The mesh size is preferably about 0.1 to 0.8 µm, and more preferably about 0.2 to 0.5 µm.

At the time of the transfusion, a reservoir needle section 273 of the connector 27 of the transfusion tube 1b is made to pierce a rubber stopper of a bag (not shown) in which a medicinal liquid containing a carcinostatic agent is reserved. As a result, the bag and the transfusion tube 1b are connected to each other through the reservoir needle section 273. Incidentally, the medicinal liquid may be preliminarily reserved in the bag, or the medicinal liquid may be stored into the bag afterwards.

Now, each of the connectors will be described below.
The upstream connector 3a has a main tube 31 and the branch tube 32 branched from the main tube 31.

The main tube 31 is disposed coaxially with the tube 21, and is connected to an intermediate portion of the tube 21. In addition, the branch tube 32 is projecting to a lateral side of the main tube 31, in other words, to a lateral side of the tube 21. The filterless transfusion tube side connector 2a of the transfusion tube 1a is detachably connected to an end portion of the branch tube 32. In other words, the end portion of the branch tube 32 constitutes a connection port. Incidentally, of the upstream connector 3a, the lumen of the main tube 31 and the branch tube 32 will be described in detail later.

The downstream connector 3b has a main tube 31 and a branch tube 32 branched from the main tube 31.

The main tube 31 is disposed coaxially with the tube 21, and is connected to the tube 21. Besides, the branch tube 32 is projecting to a lateral side of the main tube 31, in other words, to a lateral side of the tube 21. The filtered transfusion tube side connector 2b of the transfusion tube 1b is detachably connected to an end portion of the branch tube 32. In other words, the end portion of the branch tube 32 constitutes a connection port. Incidentally, of the downstream connector 3b, the lumen of the main tube 31 and the branch tube 32 will be described in detail later.

In addition, the downstream connector 3b is provided with a filter mesh 12 having a plurality of openings, or being mesh-like in shape, as a filter member for removing a foreign matter present in the liquid flowing through the flow path in the tube 21. The filter mesh 12 is disposed at a part, upstream of the branch tube 32, of the main tube 31; in the configuration shown, the filter mesh 12 is disposed at the upstream end of the main tube 31. This ensures that the branch tube 32 of the downstream connector 3b is located at a downstream side of the filter mesh 12, and the branch tube 32 of the upstream connector 3a is located at an upstream side of the filter mesh 12.

Besides, a mesh size of the filter mesh 12 is set to be smaller than the mesh size of the filter 26. Specifically, the mesh size of the filter mesh 12 is preferably about 50 to 800 µm, and more preferably about 100 to 500 µm. This ensures that the foreign matter present in the liquid flowing through the flow path in the tube 21 can be removed while keeping a flow resistance at a comparatively low value.

Here, in the transfusion tube set 100, in the case of using the transfusion tube 1a and performing the transfusion by connecting the transfusion tube 1a to the transfusion tube 1, the transfusion tube 1a is connected to the upstream connector 3a of the transfusion tube 1. On the other hand, in the case of using the transfusion tube 1b and performing the transfusion by connecting the transfusion tube 1b to the transfusion tube 1, the transfusion tube 1b is connected to the downstream connector 3b of the transfusion tube 1. In other words, the filterless transfusion tube side connector 2a of the transfusion tube 1a is detachably connected to the branch tube 32 of the upstream connector 3a of the transfusion tube 1, and the filtered transfusion tube side connector 2b of the transfusion tube 1b is detachably connected to the branch tube 32 of the downstream connector 3b of the transfusion tube 1.

This ensures that in the case of performing the transfusion by use of the transfusion tube 1a, the filter mesh 12 is intermediately present in the flow path for the medicinal liquid, whereas in the case of conducting the transfusion by use of the transfusion tube 1b, the filter mesh 12 is not intermediately present in the flow path for the medicinal liquid.

The reason for adoption of these configurations is as follows. Since the transfusion tube 1b is equipped with the filter 26, during the transfusion the foreign matter as well as the bacteria present in the medicinal liquid flowing through the flow path can be removed by the filter 26. In this case, therefore, it is unnecessary to intermediately disposing the filter mesh 12 in the flow path. In addition, the absence of the filter mesh 12 intermediately in the flow path ensures that the flow resistance during the flow of the medicinal liquid through the flow path can be reduced, as compared with the case where the filter mesh 12 is intermediately present in the flow path.

On the other hand, since the transfusion tube 1a is not equipped with the filter 26, during the transfusion the filter mesh 12 is intermediately disposed in the flow path so that the foreign matter present in the medicinal liquid flowing through the flow path is removed by the filter mesh 12.

In the transfusion tube set 100, the upstream connector 3a and the downstream connector 3b are so configured that they can be discernible on a visual basis. Besides, the filterless transfusion tube side connector 2a and the filtered transfusion tube side connector 2b are so configured that they can be distinguishable on a visual basis.

Specifically, the upstream connector 3a and the filterless transfusion tube side connector 2a are so configured that it is visually discernible that they correspond to each other, or constitute a pair. In addition, the downstream connector 3b and the filtered transfusion tube side connector 2b are so configured that it is visually distinguishable that they correspond to each other, or constitute a pair.

In this embodiment, the upstream connector 3a and the filterless transfusion tube side connector 2a have the same color, whereas the downstream connector 3b and the filtered transfusion tube side connector 2b have the same color, which is different from the color of the upstream connector 3a and the filterless transfusion tube side connector 2a. This ensures that in the case of connecting the transfusion tube 1a to the transfusion tube 1 and in the case of connecting the transfusion tube 1b to the transfusion tube 1, mis-connection can be prevented from occurring. Thus, the filterless transfusion tube side connector 2a can be connected to the upstream connector 3a, and the filtered transfusion tube side connector 2b can be connected to the downstream connector 3b, in an assured manner.

Incidentally, in each of the upstream connector 3a, the filterless transfusion tube side connector 2a, the downstream connector 3b and the filtered transfusion tube side connector 2b, the part having the same color as above-mentioned may be a part of the relevant connector or the whole part of the connector.

In addition, the configuration wherein the connectors are discernible by color as above-mentioned is not restrictive; for example, a configuration may be adopted wherein the connectors may be discernible by a shape. In a specific example, the upstream connector 3a and the filterless transfusion tube side connector 2a are provided with markers of the same shape, whereas the downstream connector 3b and the filtered transfusion tube side connector 2b are provided with markers of the same shape which is different from the shape of the markers provided for the upstream connector 3a and the filterless transfusion tube side connector 2a.

Now, the filterless transfusion tube side connector (female connector) 2a of the transfusion tube 1a and the branch tube (male connector) 32 of the upstream connector 3a of the transfusion tube 1 will be described below.

In this transfusion tube set 100, in the case of performing the transfusion by use of the transfusion tube 1a, the filterless transfusion tube side connector 2a and the upstream connector 3a are moved relative to each other, whereby the branch tube 32 of the upstream connector 3a is inserted into the filterless transfusion tube side connector 2a from the distal side of the latter, resulting in a connected state (the state shown in each of FIGS. 6 and 10) in which these connectors are connected to each other.

As shown in FIGS. 3 to 6, the filterless transfusion tube side connector 2a includes a first connector main body composed of an outer tube 4 and an inner tube 7 which are hollow cylindrical in shape, a hollow needle 5 supported by the outer tube 4, a first sealing member 6 supported by the inner tube 7, a coil spring 8 as a biasing member for biasing the first sealing member 6 in a distal direction, and a nipping member 9 disposed on the outer tube 4.

As shown in FIGS. 2 and 3, the outer tube 4 is in the shape of a bottomed tube. The branch tube 32 of the upstream connector 3a is inserted via a distal opening of the outer tube 4.

In addition, a bottom part 41 of the outer tube 4 is formed in a central area thereof with a connection section 42 which is tubular in shape and concentric with the outer tube 4. The connection section 42 is capable of supporting a proximal portion of the hollow needle 5 on a distal portion thereof. Thus, the connection section 42 communicates with the hollow needle 5. Besides, the tube 21 is connected to a proximal portion of the connection section 42. This ensures that the flow path in the tube 21 and the lumen (first flow path 52) of the hollow needle 5 communicate with each other through the connection section 42.

As shown in FIGS. 7 to 10, a wall part of the outer tube 4 is formed in an intermediate portion thereof with a groove 48 penetrating the wall part. The groove 48 is in the shape of capital "L" in a side view. The groove 48 is composed of a transverse groove 481 formed along a circumferential direction of the wall part of the outer tube 4, and a longitudinal groove 482 formed to extend proximally along a axial direction of the outer tube 4 from one end of the transverse groove 481. A projection 76 of the inner tube 7 is inserted in the groove 48. Further, the projection 76 of the inner tube 7 can move within the groove 48.

As shown in FIGS. 3 to 6, the wall part of the outer tube 4 is formed at a distal portion thereof with a pair of grooves 43, which are formed on opposite sides of the center axis of the outer tube 4. In the groove 43, two annular nipping members 9 are inserted in a stacked state. Each of the nipping members 9 functions as a part of a stopper 17 which restrains distal movement of the branch tube 32 (second connector main body 10) of the upstream connector 3a within the outer tube 4. As the configuration of the stopper 17, known configurations (for example, a configuration of a "hub attachment/detachment mechanism" described in Japanese Patent Laid-Open No. H08-126630) can be used.

In this case, each of the nipping members 9 is provided at a part of an outer circumferential portion thereof with an operating section 92 for operating and pressing the nipping member 9. With the operating section 92 pressed, the nipping member 9 is moved in a direction orthogonal to the axis of the outer tube 4.

In addition, each of the nipping members 9 is provided, at its part on the side opposite to the operating section 92, with a plurality of projections (first engaging section) 91 projecting toward the inner side. The projections 91 of the nipping member 9 on one side and the projections 91 of the nipping member 9 on the other side are disposed to be opposed to each other, with the center axis of the outer tube 4 therebetween.

Furthermore, each of the nipping members 9 is provided, on the same side as the projections 91, with a pair of elastic pieces 93 projecting from an outer circumferential surface thereof. The elastic pieces 93 of the nipping member 9 on one side are in contact with the inside of the operating section 92 of the nipping member 9 on the other side. Similarly, the elastic pieces 93 of the nipping member 9 on the other side are in contact with the inside of the operating section 92 of the nipping member 9 on the one side.

At the time of operating and pressing each of the nipping members 9, the pressing operation is conducted against a biasing force (elastic force) of the elastic pieces 93. This operation results in a state wherein the projections 91 of the nipping member 9 on one side and the projections 91 of the nipping member 9 on the other side are spaced from each other. Then, when the pressing force exerted on each of the nipping members 9 is released, the biasing forces of the elastic pieces 93 produce a state in which the projections 91 of the nipping member 9 on one side and the projections 91 of the nipping member 9 on the other side are close to each other.

In the state wherein the projections 91 of the nipping member 9 on one side and the projections 91 of the nipping member 9 on the other side are close to each other, the projections 91 are collectively engaged with an engaging section (second engaging section) 105a or 105b of the branch tube 32 of the upstream connector 3a (see FIGS. 4 to 6). This makes it possible to securely prevent a second connector 3 from being disengaged from the outer tube 4 in an unwilling manner.

Besides, in the state wherein the projections 91 of the nipping member 9 on one side and the projections 91 of the nipping member 9 on the other side are spaced from each other, the engagement between each of the nipping members 9 and the branch tube 32 of the upstream connector 3a is released.

As shown in FIG. 2, the engaging sections 105a and 105b of the branch tube 32 of the upstream connector 3a are each composed of a flange section which is formed at an outer circumferential portion of the second connector main body 10 and which is enlarged in outside diameter. The engaging sections 105a and 105b are disposed at positions spaced from each other along the axial direction of the second connector main body 10. As shown in FIGS. 4 and 6, according to a depth of insertion of the branch tube 32 of the upstream connector 3a into the filterless transfusion tube side connector 2a, one of the engaging sections 105a and 105b is engaged with the projections 91, as above-mentioned.

In the transfusion tube set 100, it can be said that the nipping members 9 and the engaging sections 105a and 105b of the branch tube 32 of the upstream connector 3a constitute the "stopper 17" by which the outer tube 4 and the branch tube 32 are locked to each other.

As shown in FIGS. 3 and 7, the wall part of the outer tube 4 is formed, at a portion of an inner circumferential part 47 thereof between the groove 48 and the groove 43, with plural (in this embodiment, four) stepped sections 49 projecting toward the inner side. As shown in FIG. 3, the inner tube 7 put in contact with the stepped sections 49, distal movement of the inner tube 7 can be restrained, so that the inner tube 7 can be securely prevented from being disengaged from the outer tube 4.

As shown in FIG. 3 (and in FIGS. 4 to 6, as well), the inner tube 7 is disposed (supported) inside the outer tube 4. The inner tube 7 is displaceable relative to the outer tube 4; specifically, the inner tube 7 is turnable about the axis of the outer tube 4, and is movable along the axial direction of the outer tube 4.

The inner tube 7 has a sealing member placing section 73 on which the first sealing member 6 is to be placed. The sealing member placing section 73 is composed of a pair of annular plate-shaped sections 731 and 732 which are provided on the inside of the inner tube 7 and between which the first sealing member 6 is clamped (nipped) from upper and lower sides.

In addition, the inner tube 7 has a sliding member 74 which slides the hollow needle 5 when the inner tube 7 is displaced, and a fixing section 75 which fixes the sliding member 74. The sliding member 74 is a member which is formed in a tubular shape from an elastic material and which has a reduced diameter section 741 reduced in inside diameter. The material forming the sliding member 74 is not particularly limited. Examples of the material include elastic materials such as various rubber materials such as natural rubber, butyl rubber, isoprene rubber, butadiene rubber, styrene-butadiene rubber, silicone rubbers, etc., various thermoplastic elastomers based on polyurethane, polyester, polyamide, olefin, styrene or the like, and mixtures of them. When the inner tube 7 is displaced, the reduced diameter section 741 makes contact with an outer circumferential part 54 of the hollow needle 5, and slides the hollow needle 5. The fixing section 75 is a tubular section formed to project integrally from the plate-shaped section 732 downward in FIG. 3.

As shown in FIG. 7 (and in FIGS. 8 to 10, as well), the wall part of the inner tube 7 is formed with a projection 76 projecting from an outer circumferential portion thereof. The projection 76 is inserted in the groove 48 in the outer tube 4, and is moved within the groove 48 as the inner tube 7 is displaced. This ensures that the filterless transfusion tube side connector 2a can assume a first state in which the projection 76 is located in the transverse groove 481 (the state shown in FIGS. 7 and 8), a second state which is reached by rotating the inner tube 7 relative to the outer tube 4 starting from the first state and in which the projection 76 is located in an intersection region 483 of the transverse groove 481 and the longitudinal groove 482 (the state shown in FIG. 9), and a third state which is reached by pushing in the inner tube 7 relative to the outer tube 4 starting from the second state and in which the projection 76 is located in the longitudinal groove 482 (the state shown in FIG. 10).

When the filterless transfusion tube side connector 2a and the upstream connector 3a are relatively moved and the branch tube 32 of the upstream connector 3a is thereby inserted into the filterless transfusion tube side connector 2a in the first state shown in FIG. 7 (FIG. 3) (hereinafter, this operation will be referred to as "the inserting operation"), a second sealing member 11 of the branch tube 32 comes into contact with the first sealing member 6 of the filterless transfusion tube side connector 2a, and attempts to push the first sealing member 6 in the proximal direction so as to move the first sealing member 6 together with the inner tube 7. Since the projection 76 of the inner tube 7 is located in the transverse groove 481 of the outer tube 4, however, proximal movement of the inner tube 7 is restrained (see FIGS. 4 and 8).

When the inner tube 7 is rotated in the direction of arrow in FIG. 9 by, for example, putting a finger on the projection 76 of the inner tube 7 starting from the state shown in FIG. 8, the filterless transfusion tube side connector 2a is put into the second state shown in FIG. 9. As a result, the restraint on the proximal movement of the inner tube 7 is released, and the movement is enabled, so that the inserting operation can be restarted. Incidentally, as shown in FIG. 5, even in the second state, a close contact state between the first sealing member 6 and the second sealing member 11 is maintained. In addition, in the second state, the inner tube 7 and the branch tube 32 (second connector main body 10) of the upstream connector 3a are locked (connected) to each other by locking means 19. This ensures that a close contact state between a first pierceable part 61 of the first sealing member 6 and a second pierceable part 111 of the second sealing member 11 is fixed. The locking means 19 will be described later.

When the branch tube 32 of the upstream connector 3a is pushed along the proximal direction against the biasing force of the coil spring 8 starting from the state shown in FIG. 9, the inserting operation is restarted, and the filterless transfusion tube side connector 2a is put into the third state shown in FIG. 10. Incidentally, as shown in FIG. 6, even in the third state, the close contact state between the first sealing member 6 and the second sealing member 11 is maintained.

When the branch tube 32 of the upstream connector 3a is withdrawn from the filterless transfusion tube side connector 2a by relatively moving the filterless transfusion tube side connector 2a and the upstream connector 3a (hereinafter, this operation will be referred to as "the withdrawing operation") in the state shown in FIG. 10 (connected state), the inner tube 7 is moved in the distal direction together with the branch tube 32 according to the biasing force of the coil spring 8, reversely to the above-mentioned, with the result that the filterless transfusion tube side connector 2a is put into the second state shown in FIG. 9. In this second state, a further distal movement of the projection 76 of the inner tube 7 is restrained. This ensures that the withdrawing operation can be once restrained in its course.

Furthermore, when the inner tube 7 is rotated in the direction reverse to the above-mentioned starting from the second state, the filterless transfusion tube side connector 2a is put into the first state shown in FIG. 8. As a result, the locked state of the inner tube 7 and the branch tube 32 of the upstream connector 3a by the locking means 19 is released, whereby distal movement of only the branch tube 32 of the upstream connector 3a is enabled, so that the withdrawing operation can be resumed. When the withdrawing operation is restarted, the filterless transfusion tube side connector 2a and the branch tube 32 of the upstream connector 3a are again put into a separated state shown in FIG. 7.

Thus, in the transfusion tube set 100, restraint on the inserting operation, release of the restraint on the inserting operation, restraint on the withdrawing operation, and release of the restraint on the withdrawing operation are carried out according to the position of the projection 76 of the inner tube 7 within the groove 48 of the outer tube 4. Therefore, the projection 76 of the inner tube 7 and the groove 48 in the outer tube 4 can be said to constitute "operation restraining means 18" which restrains these operations.

As shown in FIG. 3, the inner tube 7 is formed at the distal portion thereof with plural (in this embodiment, four) engaging pieces (elastic pieces) 77 which are projecting in the distal direction. Each of the engaging pieces 77 is provided at a distal portion thereof with pawls 771 capable of engaging with recesses (engaging section) 101a of the branch tube 32 of the upstream connector 3a.

Incidentally, the recesses 101a are a section formed in an annular shape along the circumferential direction, at a distal portion of an outer circumferential part 101 of the second connector main body 10.

The engaging pieces 77 are inclined outward in the state in which the branch tube 32 of the upstream connector 3a is not yet inserted in the filterless transfusion tube side connector 2a. As a result, in the state wherein the branch tube 32 is inserted in the filterless transfusion tube side connector 2a, each of the engaging pieces 77 can assume a state of being spaced from each recess 101a of the branch tube 32 (the state shown in FIGS. 4 and 11) and a state of approaching and engaging with the recess 101a by being pressed by pressing sections 471 of the outer tube 4 (the state shown in FIGS. 5, 6 and 12). By this engagement, the inner tube 7 and the branch tube 32 of the upstream connector 3a are assuredly locked to each other.

Incidentally, the pressing sections 471 are composed of plural (in this embodiment, four) ribs formed at an inner circumferential part 47 of the outer tube 4 along the axial direction.

In addition, the pressing sections 471 are arranged at regular intervals along the circumferential direction of the outer tube 4. In the state shown in FIG. 11, one sheet of engaging piece 77 is located between the adjacent pressing sections 471, so that pressing of the engaging pieces 77 by the pressing sections 471 is not yet performed. In this instance, the filterless transfusion tube side connector 2a is in the above-mentioned first state.

The engaging pieces 77 are arranged at regular intervals around the axis of the inner tube 7. With the inner tube 7 rotated as above-mentioned starting from the state shown in FIG. 11, each of the engaging pieces 77 climbs up an inclined surface 472 of each pressing section 471, resulting in that one pressing section 471 presses one engaging piece 77 against the elastic force of the latter. This ensures that the four engaging pieces 77 are engaged uniformly along the circumferential direction of the branch tube 32 of the upstream connector 3a, so that the inner tube 7 and the branch tube 32 are locked to each other more assuredly. In this instance, the filterless transfusion tube side connector 2a is in the above-mentioned second state. The state in which the pressing sections 471 are pressing the engaging pieces 77 is maintained even after the filterless transfusion tube side connector 2a is put into the above-mentioned third state.

Thus, in the transfusion tube set 100, it can be said that the engaging pieces 77 of the inner tube 7, the pressing sections 471 of the outer tube 4, and the recesses 101a in the branch tube 32 of the upstream connector 3a constitute the "locking means 19" by which the inner tube 7 and the branch tube 32 are assuredly locked to each other. Besides, this locking means 19 operates when the filterless tube side connector 2a is shifted from the first state into the second state; in other words, the locking means 19 operates in conjunction with a releasing operation by which the restraint on the inserting operation is released. In addition, the locking means 19 operates also when the filterless transfusion tube side connector 2a is shifted from the second state into the first state, reversely to the above; in other words, the locking means 19 operates also in conjunction with a releasing operation by which the restraint on the withdrawing operation is released. As a result, during when the filterless transfusion tube side connector 2a is displaced between the first state and the second state, or before and after the hollow needle 5 pierces the first sealing member 6 and the second sealing member 11, the close contact (liquid-tightness) between the first sealing member 6 and the second sealing member 11 can be maintained. In addition, at the time of a withdrawing operation, unexpected withdrawal of only the branch tube 32 of the upstream connector 3a (namely, disengagement of the branch tube 32) can be prevented from occurring.

Incidentally, the materials forming the outer tube 4, the inner tube 7 and the nipping members 9 are not specifically restricted. As examples of the materials, various resins may be mentioned, among which preferred are such resins as polypropylene, cyclic polyolefins and polyesters, since they are easy to mold and are low in water vapor permeability.

As shown in FIG. 3, the hollow needle 5 formed from a metallic material is disposed on the axis of the outer tube 4. As above-mentioned, the hollow needle 5 has its proximal portion supported by the connection section 42 of the outer tube 4.

The hollow needle 5 is tubular in shape, with its lumen functioning as the first flow path 52 which permits a medicinal liquid (liquid) to pass therethrough. In addition, the hollow needle 5 is closed at its distal end, and has a side hole (opening) 53 opened in a distal portion of the wall part thereof. The side hole 53 communicates with the first flow path 52.

The hollow needle 5 is formed with a sharp needlepoint 51 at the distal end thereof. As shown in FIG. 6, the needlepoint 51 can pierce the first sealing member 6 of the filterless transfusion tube side connector 2a and the second sealing member 11 of the branch tube 32 of the upstream connector 3a which will be described later. As shown in FIG. 6, in the connected state, that portion of the hollow needle 5 which ranges from the needlepoint 51 to the part where the side hole 53 is formed is exposed (projected) to the inside of the lumen of the branch tube 32. This ensures that the lumen of the hollow needle 5 and the lumen of the branch tube 32 communicate with each other, in other words, the first flow path 52 in the filterless transfusion tube side connector 2a and a second flow path 102 in the branch tube 32 (described later) communicate with each other, through the side hole 53 of the hollow needle 5.

As shown in FIG. 3, the first sealing member 6 is disposed inside the inner tube 7. The first sealing member 6, which seals off a lumen part of the inner tube 7, is circular disk-like in shape and is so disposed that its thickness direction coincides with the axial direction of the inner tube 7. This ensures that the first sealing member 6 is easily and assuredly pierced through by the needlepoint 51 of the hollow needle 5 when it is moved toward the proximal side along the axial direction of the hollow needle 5.

In addition, the first sealing member 6 is an elastic body having a thickness of which is greater at a central portion than at an edge portion. The central portion is the first pierceable part 61 to be pierced through by the hollow needle 5. Besides, the edge portion of the first sealing member 6 is nipped by the pair of plate-shaped sections 731 and 732 of the inner tube 7, as above-mentioned. This ensures that the first sealing member 6 is assuredly fixed to the inner tube 7, and can be moved together with the inner tube 7.

In addition, a sum total of a sliding resistance between those parts of the first sealing member 6 (first pierceable part 61) and the second sealing member 11 (second pierceable part 111) which are pierced through by the hollow needle 5 and that outer circumferential part 54 of the hollow needle 5 which makes contact with the parts and a sliding resistance between the reduced diameter section 741 of the sliding member 74 and that outer circumferential part 54 of the hollow needle 5 which makes contact with the section, is preferably set to be smaller than the biasing force of the coil spring 8. Such a setting ensures that when the filterless transfusion tube side connector 2a has released the stopper 17 being in the third state shown in FIG. 6, the filterless transfusion tube side connector 2a can be returned into the second state shown in FIG. 2 by the biasing force of the coil spring 8. Incidentally, a method for setting the magnitude relations between these forces is not specifically restricted. Examples of the method include a method of selecting the materials for forming the first sealing member 6, the second sealing member 11 and the sliding member 74, a method of controlling the thicknesses of the first pierceable part 61 and the second pierceable part 111, a method of selecting the material for forming the coil spring 8 and/or controlling a wire diameter or a number of winding of the coil spring 8, and a method of controlling the outside diameter of the hollow needle 5.

As shown in FIG. 3, in a natural state where no external force is exerted, a distal end face 612 of the first pierceable part 61 is protuberant. In the close contact state where the sealing member 6 and the second sealing member 11 are in close contact with each other, as shown in FIG. 4, the distal end face 612 having been protuberant is flattened. This ensures that the close contact state becomes more assured, so that the liquid-tightness at a boundary between the first sealing member 6 and the second sealing member 11 can be secured. Consequently, transfer of the medicinal liquid in the connected state can be performed safely and reliably.

Incidentally, the material forming the first sealing member 6 is not specifically restricted. For example, the same materials as those mentioned above as examples of the material for forming the sliding member 74 can be used.

As shown in FIG. 3, the coil spring 8 formed of a metallic material such as stainless steel is disposed inside the outer tube 4. The coil spring 8, in a compressed state, has its distal end in contact with the plate-shaped section 732 of the inner tube 7 and has its proximal end in contact with the bottom part 41 of the outer tube 4. This ensures that the first sealing member 6 can be securely biased in the distal direction through the inner tube 7. Incidentally, as the biasing means, not only the coil spring 8 but also a bellows-like leaf spring and hollow cylindrical or bellows-like rubber members may be used.

As shown in FIGS. 2 and 3, the branch tube 32 of the upstream connector 3a includes the tubular second connector main body 10 and the second sealing member 11 provided on the second connector main body 10.

The second connector main body 10 is a hollow cylindrical member. A lumen of the second connector main body 10 functions as the second flow path 102 which permits liquid to pass therethrough.

In addition, the lumen of the main tube 31 of the upstream connector 3a described above is separated into an upstream part and a downstream part. The upstream part and the downstream part communicate with each other via the second flow path 102.

Specifically, the second flow path 102 includes two unit flow paths 102a and 102b which communicate with each other at a proximal part of the second connector main body 10. The unit flow path 102a on one side communicates with an upstream part of the lumen of the main tube 31, whereas the unit flow path 102b on the other side communicates with a downstream part of the lumen of the main tube 31.

Besides, as above-mentioned, the second connector main body 10 is formed at an intermediate portion thereof with engaging sections 105a and 105b for engagement with the first engaging section 91 of the filterless transfusion tube side connector 2a.

In addition, the second connector main body 10 is formed at an outer circumferential portion thereof with plural (in this embodiment, four) ribs 104 extending along a longitudinal direction thereof. These ribs 104 are arranged at regular intervals along the circumferential direction of the outer circumferential portion of the second connector main body 10. With this structure, the second connector main body 10 can be reinforced.

The second connector main body 10 is provided at a distal portion thereof with a sealing member placing section 106 on which the second sealing member 11 is to be placed. The sealing member placing section 106 is composed of a pair of annular plate-shaped parts 106a and 106b between which the second sealing member 11 is clamped (nipped) from upper and lower sides.

Incidentally, the material forming the second connector main body 10 is not particularly limited. For example, such materials as mentioned above in the description of the outer tube 4, the inner tube 7 and the nipping members 9 of the filterless transfusion tube side connector 2a can be used.

As shown in FIG. 3, the second sealing member 11, which seals off the lumen part of the second connector main body 10, is circular disk-like in shape and is so disposed that its thickness direction coincides with the axial direction of the second connector main body 10. This ensures that the second sealing member 11 is easily and assuredly pierced through by the needlepoint 51 of the hollow tube 5, together with the first sealing member 6 in close contact therewith.

In addition, the second sealing member 11 is an elastic body having a thickness of which is greater at a central portion than at an edge portion. The central portion is the second pierceable part 111 to be pierced through by the hollow needle 5. Besides, the edge portion of the second sealing member 11 is nipped by the pair of plate-shaped sections 106a and 106b of the second connector main body 10, as above-mentioned. This ensures that the second sealing member 11 is assuredly fixed to the second connector main body 10.

As shown in FIG. 3, in a natural state where no external force is exerted, a proximal end face 112 of the second pierceable part 111 is protuberant. In the close contact state where the first sealing member 6 and the second sealing member 11 are in close contact with each other, as shown in FIG. 4, the proximal end face 112 having been protuberant is flattened, in the same manner as the distal end face 612 of the first sealing member 6. This ensures that the close contact state becomes more assured, so that the liquid-tightness at a boundary between the first sealing member 6 and the second sealing member 11 can be secured.

Incidentally, the material forming the second sealing member 11 is not specifically restricted. For example, the same materials as those mentioned above as examples of the material for forming the sliding member 74 can be used.

Now, the filtered transfusion tube side connector 2b and the downstream connector 3b will be described below.

The configuration of the filtered transfusion tube side connector 2b is the same as that of the filterless transfusion tube side connector 2a described above. Specifically, the filtered transfusion tube side connector 2b includes a third connector main body composed of an outer tube 4 and an inner tube 7 which are hollow cylindrical in shape, a hollow needle 5 supported by the outer tube 4, a third sealing member having a third pierceable part supported by the inner tube 7, a coil spring 8 as a biasing member for biasing the third sealing member in the distal direction, and a nipping member 9 disposed on the outer tube 4. Incidentally, configurations of the third connector main body and the third sealing member having the third pierceable part are respectively the same as those of the first connector main body and the first sealing member 6 having the first pierceable part 61 of the filterless transfusion tube side connector 2a described above.

In the following, description of the filtered transfusion tube side connector 2b will be omitted. For the filtered transfusion tube side connector 2b, the above description of the filterless transfusion tube side connector 2a is to be diverted while reading "first" in the names of component members or parts as "third."

In addition, the configurations of a lumen of a main tube 31 and a branch tube 32 in a downstream connector 3b are the same as those in the upstream connector 3a described above. Specifically, the branch tube 32 of the downstream connector 3b includes a tubular fourth connector main body 13, and a fourth sealing member having a fourth pierceable part provided on the fourth connector main body 13. Incidentally, configurations of the fourth connector main body 13 and the fourth sealing member having the fourth pierceable part are respectively the same as those of the second connector main body 10 and the second sealing member 11 having the second pierceable part 111 of the upstream connector 3a described above.

In the following, description of the downstream connector 3b will be omitted. For the downstream connector 3b, the above description of the upstream connector 3a is to be diverted while reading "second" in the names of the component members or parts as "fourth".

Now, an example of the procedure of using the transfusion tube set 100 will be described below.
In the first place, in the case of performing the transfusion by use of the transfusion tube 1, for example, in the case of administering a medicinal liquid containing an antiemetic or the like for preventing or restraining side effects of a carcinostatic agent, first, a rubber stopper of a bag (not shown) in which the medicinal liquid has preliminarily been reserved is pierced through by the reservoir needle 22 of the transfusion tube 1. As a result, the bag and the transfusion tube 1 are connected to each other through the reservoir needle 22.

Incidentally, a connector (not shown) on the downstream side of the transfusion tube 1 is in connection with a connector for an indwelling needle set indwelling in a patient's blood vessel.

Next, the medicinal liquid is administered while controlling the flow rate of the medicinal liquid by operating the roller 242 of the roller clamp 24 on the transfusion tube 1. The medicinal liquid flows downstream from the bag through the flow path in the transfusion tube 1 and through the filter mesh 12, to be administered into the patient. In this case, even if there is foreign matter mixed in the medicinal liquid, the foreign matter can be trapped by the filter mesh 12 and can therefore be removed.

The transfusion tube 1a is used in such cases as a case where there is no fear of mixing of bacteria into the medicinal liquid or a case where a medicine contained in the medicinal liquid would be trapped by the filter 26 in the transfusion tube 1b.

In the case of performing the transfusion by use of the transfusion tube 1a, specifically, in the case of administering a medicinal liquid containing a carcinostatic agent by connecting the transfusion tube 1a to the transfusion tube 1, a bag (not shown) in which the medicinal liquid has preliminarily been reserved is used. In this case, there is no fear of mixing of bacteria into the medicinal liquid.

First, a rubber stopper of the bag is pierced through by the reservoir needle section 273 of the connector 27 of the transfusion tube 1a. As a result, the bag and the transfusion tube 1a are connected to each other through the reservoir needle section 273.

Next, priming of the transfusion tube 1a is conducted. By these operations, preparation on the transfusion tube 1a side is completed.

Subsequently, the transfusion tube 1a is connected to the transfusion tube 1. Specifically, the filterless transfusion tube side connector 2a of the transfusion tube 1a is connected to the branch tube 32 of the upstream connector 3a of the transfusion tube 1.

Now, the operating states of the filterless transfusion tube side connector 2a and the upstream connector 3a in the case where the transfusion tube 1a is connected to the transfusion 1, in other words, the process in which the filterless transfusion tube side connector 2a and the upstream connector 3a are shifted from a separated state into a connected state (refer to the drawings in the order of FIG. 3 (FIG. 7) → FIG. 4 (FIG. 8) → FIG. 5 (FIG. 9) → FIG. 6 (FIG. 10)) will be described below.

First, as shown in FIG. 3, the filterless transfusion tube side connector 2a in the separated state is made to approach a proximal portion of the branch tube 32 of the upstream connector 3a, with its distal end ahead. In the separated state, the filterless transfusion tube side connector 2a is in the first state (the state wherein the projection 76 of the inner tube 7 is located in the transverse groove 481 of the groove 48 in the outer tube 4) (see FIG. 7). In addition, the first sealing member 6 is located on the distal side relative to the hollow needle 5.

As the branch tube 32 of the upstream connector 3a is gradually inserted into the filterless transfusion tube side connector 2a, as shown in FIGS. 4 and 8, first, the distal end face 612 of the first sealing member 6 of the filterless transfusion tube side connector 2a and the proximal end face 112 of the second sealing member 11 of the second connector 3 are brought into contact with each other and are elastically deformed to make close contact with each other. In this instance, since the filterless transfusion tube side connector 2a is in the first state (see FIG. 8) as above-mentioned, the inserting operation of inserting the branch tube 32 of the upstream connector 3a into the filterless transfusion tube side connector 2a is once restrained.

In addition, as shown in FIG. 4, the stopper 17 operates (the nipping member 9 of the filterless transfusion tube side connector 2a is engaged with the engaging section 105a of the branch tube 32 of the upstream connector 3a), whereby the branch tube 32 is prevented from being again moved back in the distal direction to be disengaged from the filterless transfusion tube side connector 2a.

Next, when the inner tube 7 of the filterless transfusion tube side connector 2a is rotated in the direction of arrow, as shown in FIG. 9, the filterless transfusion tube side connector 2a is put into the second state (the state wherein the projection 76 of the inner tube 7 is located in the intersection region 483 of the groove 48 in the outer tube 4). Consequently, as above-mentioned, the restraint on the inserting operation is released, so that the inserting operation can be restarted.

Besides, the connected state of the filterless transfusion tube side connector 2a and the branch tube 32 of the upstream connector 3a is maintained by the stopper 17 and the locking means 19. This makes it possible to securely prevent the branch tube 32 of the upstream connector 3a from being withdrawn from the filterless transfusion tube side connector 2a, in other words, to securely prevent the filterless transfusion tube side connector 2a and the upstream connector 3a in the connected state from being unwillingly separated from each other. Consequently, the medicinal liquid can be safely transferred via the filterless transfusion tube side connector 2a and the upstream connector 3a. In other words, deposition of the medicinal liquid on a health care staff can be prevented from occurring.

Besides, in the connected state, the close contact between the first sealing member 6 of the filterless transfusion tube side connector 2a and the second sealing member 11 of the branch tube 32 of the upstream connector 3a is maintained (see FIG. 6). As a result, the liquid-tightness (air-tightness) of the first flow path 52 and the second flow path 102, particularly in the vicinity of the joint between the flow paths, can be securely maintained. Thus, the medicinal liquid passing through these flow paths can be securely prevented from leaking from any of the filterless transfusion tube side connector 2a and the upstream connector 3a in the connected state. Accordingly, the deposition of the medicinal liquid on the health care staff can be prevented from occurring.

In addition, in the state shown in FIG. 6, a proximal end 78 of the inner tube 7 and a distal end 412 of a wall part 411 rising from the bottom part 41 of the outer tube 4 make contact with each other. This restrains (determines) the limit of insertion of the second connector 3.

Subsequently, the administration of the medicinal liquid is started while controlling the flow rate of the medicinal liquid by operating the roller 242 of the roller clamp 24 on the transfusion tube 1a. The medicinal liquid flows downstream from the bag through the flow path in the transfusion tube 1a, then flows through the filterless transfusion tube side connector 2a, the branch tube 32 and the main tube 31 of the upstream connector 3a, and flows downstream through the flow path in the transfusion tube 1, to be administered into the patient via the filter mesh 12. In this case, even if there is a foreign matter mixed in the medicinal liquid, the foreign matter can be trapped by the filter mesh 12 and can therefore be removed.

When the administration of the medicinal liquid is finished, the transfusion tube 1a is detached from the transfusion tube 1. Specifically, the filterless transfusion tube side connector 2a of the transfusion tube 1a is detached from the branch tube 32 of the upstream connector 3a of the transfusion tube 1.

Now, operating states of the filterless transfusion tube side connector 2a and the upstream connector 3a in the case where the transfusion tube 1a is detached from the transfusion tube 1, in other words, the process in which the filterless transfusion tube side connector 2a and the upstream connector 3a are shifted from the connected state again into the separated state (refer to the drawings in the order of FIG. 6 (FIG. 10) → FIG. 5 (FIG. 9) → FIG. 4 (FIG. 8) → FIG. 3 (FIG. 7)) will be described below.

First, starting from the state shown in FIGS. 6 and 10, the nipping member 9 is operated so that the locked state of the filterless transfusion tube side connector 2a and the branch tube 32 of the upstream connector 3a is released. This makes it possible to start a withdrawing operation by which the branch tube 32 of the upstream connector 3a inserted in the filterless transfusion tube side connector 2a is withdrawn from the filterless transfusion tube side connector 2a.

When the withdrawing operation is started, as shown in FIGS. 5 and 9, the filterless transfusion tube side connector 2a is moved in the proximal direction, reversely to the above-mentioned. In other words, the branch tube 32 of the upstream connector 3a is moved in the distal direction. In this instance, since the biasing force of the coil spring 8 is acting on the first sealing member 6 through the inner tube 7, the first sealing member 6 can follow up to the movement of the branch tube 32 of the upstream connector 3a. This ensures that the close contact state of the first sealing member 6 and the second sealing member 11 can be maintained also during the withdrawing operation.

When the filterless transfusion tube side connector 2a is put into the second state, the withdrawing operation is once restrained (see FIG. 9), as above-mentioned. In this instance, as shown in FIG. 5, the side hole 53 of the hollow needle 5 is located on the proximal side relative to the second sealing member 11 (in the configuration shown, relative to the first sealing member 6 which is located on the more proximal side than the second sealing member 11). Incidentally, of the first sealing member 6 and the second sealing member 11, those parts having been pierced through by the hollow needle 5 are closed by their self-closing properties.

Next, the inner tube 7 is rotated in the direction reverse to the above-mentioned, whereon the filterless transfusion tube side connector 2a is put into the first state shown in FIG. 8. In this instance, as shown in FIG. 4, the locked state of the inner tube 7 and the branch tube 32 of the upstream connector 3a brought about by the locking means 19 is released, whereby distal movement of only the branch tube 32 of the upstream connector 3a is enabled. As a result, the withdrawing operation of withdrawing the branch tube 32 of the upstream connector 3a can be resumed.

When the withdrawing operation is restarted, as shown in FIGS. 3 and 7, the first pierceable part 61 and the second pierceable part 111 having been in the close contact state are spaced from each other, and the filterless transfusion tube side connector 2a and the upstream connector 3a having been in the connected state can be again put into the separated state.

Thus, in the transfusion tube set 100, at the time of withdrawing the branch tube 32 of the upstream connector 3a from the filterless transfusion tube side connector 2a, the first sealing member 6 and the second sealing member 11 can be prevented from being spaced from each other before the hollow needle 5 is withdrawn completely from the second sealing member 11. This ensures that even during when the filterless transfusion tube side connector 2a and the upstream connector 3a in the connected state are separated from each other, the liquid-tightness of the first flow path 52 and the second flow path 102 is maintained. Therefore, the medicinal liquid in the flow paths can be securely prevented from leaking from any of the filterless transfusion tube side connector 2a and the upstream connector 3a. This enables safe transfer of the medicinal liquid. In other words, the deposition of the medicinal liquid on the health care staff can be prevented from occurring.

The transfusion tube 1b is used in such cases as a case where there is a fear of mixing of bacteria into the medicinal liquid or a case where the medicine contained in the medicinal liquid would not be trapped by the filter 26.

In the case of performing the transfusion by use of the transfusion tube 1b, specifically, in the case of administering a medicinal liquid containing a carcinostatic agent by connecting the transfusion tube 1b to the transfusion tube 1, first, a syringe (not shown) in which a dissolving liquid is stored is connected to a carcinostatic agent vial (not shown) in which a powdery carcinostatic agent is stored via an adapter (not shown), and the dissolving liquid is transferred from the syringe into the carcinostatic agent vial. Thus, the carcinostatic agent is dissolved in the dissolving liquid, after which the resulting medicinal liquid containing the carcinostatic agent is sucked into the syringe.

Next, a rubber stopper of a bag (not shown) is pierced through by the reservoir needle section 273 of the connector 27 of the transfusion tube 1b. As a result, the bag and the transfusion tube 1b are connected to each other via the reservoir needle section 273. Incidentally, this operation may be conducted before an operation of storing the medicinal liquid into the syringe.

Subsequently, priming of the transfer tube 1b is carried out.
Next, the syringe is connected to the branch tube 272 of the connector 27 through an adapter, the medicinal liquid in the syringe is transferred from the syringe into the bag, after which the syringe is detached from the branch tube 272. Incidentally, during these steps, there may possibly be a fear of mixing of bacteria into the medicinal liquid; therefore, the transfusion tube 1b is used, for securing safety. By these operations, preparation on the transfusion tube 1b side is completed.

Subsequently, like in the case of the transfusion tube 1a, the filtered transfusion tube side connector 2b of the transfusion tube 1b is connected to the branch tube 32 of the downstream connector 3b of the transfusion tube 1, and administration of the medicinal liquid is performed. The medicinal liquid flows downstream from the bag through the flow path in the transfusion tube 1b, then flows through the filter 26, the filtered transfusion tube side connector 2b, and the branch tube 32 and the main tube 31 of the downstream connector 3b, and flows downstream through the flow path in the transfusion tube 1, to be administered into the patient. In this case, even if a bacteria or foreign matter has mixed into the medicinal liquid, the bacteria or foreign matter can be trapped by the filter 26 and can therefore be removed.

In addition, since the medicinal liquid does not pass through the filter mesh 12, a flow resistance during flow of the medicinal liquid through the flow paths can be reduced, as compared with the case where the medicinal liquid flows through the filter mesh 12.

When the administration of the medicinal liquid is over, the filtered transfusion tube side connector 2b of the transfusion tube 1b is detached from the branch tube 32 of the downstream connector 3b of the transfusion tube 1, like in the case of the transfusion tube 1a.

Incidentally, the operating states of the filtered transfusion tube side connector 2b and the downstream connector 3b in the case of connecting and detaching the filtered transfusion tube side connector 2b and the branch tube 32 of the downstream connector 3b to and from each other are respectively the same as those in the case of the filterless transfusion tube side connector 2a and the upstream connector 3a described above; therefore, descriptions of the operating states are omitted here.

As above-mentioned, according to this transfusion tube set 100, the health care staff can attach and detach the transfusion tubes 1a and 1b safely, without touching the medicinal liquid containing the carcinostatic agent or the like to be administered.

In addition, when the transfusion tube 1b equipped with the filter 26 is used, the filter mesh 12 is not intermediately present in the flow path for the medicinal liquid to be administered, so that the flow resistance during flow of the medicinal liquid through the flow path can be reduced, as compared with the case where the filter mesh 12 member is intermediately disposed in the flow path.

Besides, when the transfusion tube 1a not equipped with the filter 26 is used, the filter mesh 12 is intermediately disposed in the flow path for the medicinal liquid to be administered, so that the foreign matter present in the medicinal liquid flowing through the flow path can be removed.

Incidentally, while the filter mesh 12 is provided at the downstream connector 3b in the present embodiment, this configuration is not restrictive; thus, another configuration may be adopted, insofar as the filter mesh 12 is provided in the flow path between the branch tube 32 of the upstream connector 3a and the branch tube 32 of the downstream connector 3b. For example, the filter mesh 12 may be provided at the upstream connector 3a. Further, the filter mesh 12 may be provided at other part than the upstream connector 3a and the downstream connector 3b, for example, at a part between the upstream connector 3a and the downstream connector 3b.

In the case where the filter mesh 12 is provided at the upstream connector 3a, the filter mesh 12 is disposed at a part downstream of the branch tube 32 of the main tube 31, for example, at the downstream end part of the main tube 31.

### <Second Embodiment>

FIG. 13 is a plan view schematically showing a connector for a transfusion tube in a second embodiment of a transfusion tube set according to the present invention.

Incidentally, in the following, description will be made while referring to the upper side in FIG. 13 as "upstream (side)" and the lower side as "downstream (side) ".
In addition, in regard of branch tubes 332 and 342 of a connector 3 in FIG. 13, description will be made while referring to the right side as "proximal end" and the left side as "distal end", as indicated by the parenthesized words.

Now, the second embodiment will be described below. The following description will be made to center on differences from the first embodiment described above, and descriptions of the same items as above will be omitted.

The second embodiment is the same as the first embodiment, except for differences in the configuration of those connectors in a transfusion tube 1 which are to be connected to transfusion tubes 1a and 1b.

Specifically, in a transfusion tube set 100 according to the second embodiment, as shown in FIG. 13, the transfusion tube 1 has the connector 3 on a downstream side of a roller clamp 24.

This connector 3 corresponds to the upstream connector 3a as well as the downstream connector 3b in the first embodiment. The connector 3 includes an upstream connector section 33 equivalent or similar to the upstream connector 3a, and a downstream connector section 34 equivalent or similar to the downstream connector 3b. Incidentally, the downstream connector section 34 is located downstream of the upstream connector section 33.

The upstream connector section 33 includes a main tube 331 and a branch tube 332 branched from the main tube 331; similarly, the downstream connector section 34 includes a main tube 341 and a branch tube 342 branched from the main tube 341. The upstream connector section 33 and the downstream connector section 34 are united together by joining the downstream end of the main tube 331 of the upstream connector section 33 and the upstream end of the main tube 341 of the downstream connector section 34 to each other.

A filter mesh 12 is provided in a flow path between the branch tube 322 of the upstream connector section 33 and the branch tube 342 of the downstream connector section 34. Incidentally, in the configuration shown in the figure, the filter mesh 12 is provided between the downstream end of the main tube 331 of the upstream connector section 33 and the upstream end of the main tube 341 of the downstream connector section 34.

According to this transfusion tube set 100, the same effects as those in the first embodiment can be obtained.

In addition, in the transfusion tube set 100, the length of the flow path between the branch tube 332 of the upstream connector section 33 and the branch tube 342 of the downstream connector section 34 can be shortened, whereby the time required for priming can be shortened, as compared with the first embodiment.

While the transfusion tube and the transfusion tube set according to the present invention have been described above based on the embodiments shown in the drawings, the invention is not to be limited to the embodiments. The configuration of each component member or part can be replaced by an arbitrary configuration having an equivalent function. Besides, other arbitrary structures may be added to the present invention.

In addition, the present invention may be embodied as a combination of arbitrary two or more configurations (characteristic features) of the above-described embodiments.

Besides, the configurations of the upstream connector, the downstream connector, the filterless transfusion tube side connector, and the filtered transfusion tube side connector are not restricted respectively to the above-described ones, insofar as the corresponding connectors can be connected to each other.

In addition, while the operation restraining means has been configured to be composed of the groove formed in the wall part of the outer tube and the projection which is projected from the wall part of the inner tube and inserted in the groove, this configuration is not restrictive. The operation restraining means may be composed of a groove formed in the wall part of the inner tube and a projection which is projected from the wall part of the outer tube and inserted in the groove.

Besides, while the first pierceable part and the second pierceable part have respective end faces which are protuberant, this configuration is not restrictive. For example, an end face of one of these pierceable parts may only be protuberant.

In addition, while the third pierceable part and the fourth pierceable part have respective end faces which are protuberant, this configuration is not restrictive. For example, an end face of one of these pierceable parts may only be protuberant.

### Industrial Applicability

According to the present invention, a filtered transfusion tube and a filterless transfusion tube can be attached and detached safely, without touching a liquid to be administered.
In addition, when the filtered transfusion tube is used, a configuration in which a filter member is not intermediately provided in a flow path for a liquid to be administered can be adopted; besides, when the filterless transfusion tube is used, a configuration in which a filter member is intermediately provided in the flow path for the liquid to be administered can be adopted. Accordingly, the present invention has industrial applicability.

## Claims

1. A transfusion tube comprising:
a tube constituting a flow path for liquid containing a foreign matter;
a filter member which is provided at an intermediate portion of the flow path and which removes the foreign matter present in the liquid flowing through the flow path;
an upstream connector provided in the flow path upstream of the filter member; and
a downstream connector provided in the flow path downstream of the filter member,
wherein the transfusion tube is configured so that a filtered transfusion tube, which is used during transfusion and which has a filter permitting the liquid to pass therethrough but preventing bacteria from passing therethrough, is connected to the downstream connector, and whereas a filterless transfusion tube, which is used during the transfusion and which has no the filter, is connected to the upstream connector.

2. The transfusion tube according to claim 1,
wherein the upstream connector has a branch tube projected to a lateral side of the tube, and the branch tube being provided at an end portion thereof with a connection port to which the filterless transfusion tube is to be connected, and
wherein the downstream connector has a branch tube projected to a lateral side of the tube, the branch tube being provided at an end portion thereof with a connection port to which the filtered transfusion tube is to be connected.

3. The transfusion tube according to claim 2, wherein the filter mesh is provided downstream of the branch tube of the upstream connector, or upstream of the branch tube of the downstream connector.

4. The transfusion tube according to claim 1, wherein the filterless transfusion tube and the filtered transfusion tube are each for use in administering a carcinostatic agent.

5. A transfusion tube set comprising:
the transfusion tube according to claim 1;
the filtered transfusion tube which is used during the transfusion, and which has a tube constituting a flow path for the liquid and the filter provided at an intermediate portion of the flow path and permitting the liquid to pass therethrough but preventing the bacteria from passing therethrough; and
the filterless transfusion tube which is used during the transfusion, and which has a tube constituting a flow path for the liquid and no the filter,
wherein the filtered transfusion tube is connected to the downstream connector, and the filterless transfusion tube is connected to the upstream connector.

6. The transfusion tube set according to claim 5,
wherein the filterless transfusion tube has a filterless transfusion tube side connector which is connected to the upstream connector, and
the filtered transfusion tube has a filtered transfusion tube side connector which is connected to the downstream connector,
wherein one connector of the upstream connector and the filterless transfusion tube side connector includes a tubular first connector main body having an opening to insert the other connector at a distal end of the first connector main body, a hollow needle provided in a lumen part of the first connector main body so as to communicate with the flow path and having an opening at a distal portion of the follow needle, a first sealing member having a first pierceable part pierceable by the hollow needle and formed from an elastic material to seal off the lumen part of the first connector main body, and a biasing member for biasing the first pierceable part in a distal direction,
wherein the other connector includes a tubular second connector main body of which distal end communicates with the flow path, and a second sealing member provided at a proximal end of the second connector main body, having a second pierceable part making close contact with the first pierceable part when inserted into the first connector main body, and formed from an elastic material to seal off a lumen part of the second connector main body,
wherein the one connector is provided with operation restraining means by which at the time of inserting the other connector into the one connector, an inserting operation is once restrained in its course and restarting of the inserting operation is enabled when the restraint is removed, and, at the time of withdrawing the other connector from the one connector, a withdrawing operation is once restrained in its course and restarting of the withdrawing operation is enabled when the restraint is removed,
wherein in a process of inserting the other connector into the one connector, the inserting operation is once restrained by the operation restraining means when the first pierceable part and the second pierceable part are brought into a close contact state, and, when the inserting operation is restarted by removing the restraint, the first pierceable part and the second pierceable part in the close contact state are pierced through by the hollow needle, and the opening of the hollow needle is located on the distal side relative to the second pierceable part, to be exposed to the inside of the lumen part of the second connector main body, and
wherein in a process of withdrawing the other connector from the one connector, the withdrawing operation is once restrained by the operation restraining means when the opening of the hollow needle is located on the proximal side relative to the first pierceable part in the close contact state, and, when the withdrawing operation is restarted by removing the restraint, the first pierceable part and the second pierceable part are spaced away from each other.

7. The transfusion tube set according to claim 5,
wherein the filterless transfusion tube has a filterless transfusion tube side connector which is connected to the upstream connector, and
the filtered transfusion tube has a filtered transfusion tube side connector which is connected to the downstream connector,
wherein one connector of the downstream connector and the filtered transfusion tube side connector includes a tubular third connector main body having an opening to insert the other connector at a distal end of the third connector main body, a hollow needle provided in a lumen part of the third connector main body so as to communicate with the flow path and having an opening at a distal portion of the hollow needle, a third sealing member having a third pierceable part pierceable by the hollow needle and formed from an elastic material to seal off the lumen part of the third connector main body, and a biasing member for biasing the third pierceable part in a distal direction,
wherein the other connector includes a tubular fourth connector main body of which distal end communicates with the flow path, and a fourth sealing member provided at a proximal end of the fourth connector main body, having a fourth pierceable part making close contact with the third pierceable part when inserted into the third connector main body, and formed from an elastic material to seal off a lumen part of the fourth connector main body,
wherein the one connector is provided with operation restraining means by which at the time of inserting the other connector into the one connector, an inserting operation is once restrained in its course and restarting of the inserting operation is enabled when the restraint is removed, and, at the time of withdrawing the other connector from the one connector, awithdrawing operation is once restrained in its course and restarting of the withdrawing operation is enabled when the restraint is removed,
wherein in a process of inserting the other connector into the one connector, the inserting operation is once restrained by the operation restraining means when the third pierceable part and the fourth pierceable part are brought into a close contact state, and, when the inserting operation is restarted by removing the restraint, the third pierceable part and the fourth pierceable part in the close contact state are pierced through by the hollow needle, and the opening of the hollow needle is located on the distal side relative to the fourth pierceable part, to be exposed to the inside of the lumen part of the fourth connector main body, and
wherein in a process of withdrawing the other connector from the one connector, the withdrawing operation is once restrained by the operation restraining means when the opening of the hollow needle is located on the proximal side relative to the third pierceable part in the close contact state, and, when the withdrawing operation is restarted by removing the restraint, the third pierceable part and the fourth pierceable part are spaced away from each other.
